# EUROPÄISCHE PATENTSCHRIFT

(11) **EP 0 840 634 B1**
(45) Veröffentlichungstag und Bekanntmachung des Hinweises auf die Patenterteilung: **31.10.2001**
(21) Anmeldenummer: 96926348.2
(22) Anmeldetag: 15.07.1996
(51) Int. Cl.: A61M 37/00

(54) **TRANSCORNEALES ARZNEIMITTELFREIGABESYSTEM**
TRANSCORNEAL DRUG-RELEASE SYSTEM
SYSTEME TRANSCORNEEN DE LIBERATION DE MEDICAMENT

(30) Priorität: 14.07.1995 DE 19525607
(43) Veröffentlichungstag der Anmeldung: 13.05.1998
(73) Patentinhaber: Boehringer Ingelheim Pharma KG, 55218 Ingelheim am Rhein (DE)
(72) Erfinder: EICHER, Joachim, D-44227 Dortmund (DE); ZIERENBERG, Bernd, D-55411 Bingen (DE)
(86) Internationale Anmeldenummer: EP9603090
(87) Internationale Veröffentlichungsnummer: WO9703718

(56) Entgegenhaltungen:
- EP-A- 0 513 879
- DE-A- 19 518 974
- GB-A- 2 221 394
- US-A- 3 964 482

## Beschreibung

Die vorliegende Erfindung betrifft ein neues Arzneimittelfreigabesystem zur kontrollierten Abgabe von Arzneimitteln über einen längeren Zeitraum.

Erfindungsgemäß beansprucht wird ein transcomeales System zur kontrollierten Zuführung von Arzneimitteln unter Umgehung des Magen-Darm-Traktes, das im wesentlichen aus einer Vorrichtung besteht, die es ermöglicht unter Umgehung der cornealen Hautschichten ein Medikament über einen längeren Zeitraum zu applizieren.

Das Dokument US-A-3964482 beschreibt ein transcorneales System zur kontrollierten Freigabe von Arzneimitteln, welches ein Wirkstoffreservoir sowie eine Vorrichtung mit Mikrostacheln oder -schneiden umfaßt.

Die erfindungsgemäße Vorrichtung besteht im wesentlichen aus einem Reservoir für das Arzneimittel und mindestens einem - typischerweise mehreren - mit kapillaren Öffnungen versehenen Mikrostacheln, die so mit dem Reservoir in Verbindung stehen, daß das Arzneimittel in Form einer wirkstoffhaltigen Lösung aus dem Reservoir in die Mikrostacheln gelangt. Bei Aufsetzen des transcornealen Systems auf die Haut wird das Stratum corneum und gegebenenfalls die Epidermis von den Mikrostacheln durchdrungen, so daß ein direkter Zugang zur innervierten Hautschicht gegeben ist. Damit kann das Arzneimittel aus dem Reservoir durch die kapillaren Öffnungen der Mikrostacheln bis in vaskularisierte Abschnitte der Haut gelangen, um dort über das kapillare Kreislaufsystem in den Blutkreislauf aufgenommen zu werden. Anstelle der Mikrostacheln können auch Mikroschneiden verwendet werden, die dann beim Aufsetzen des Systems die Haut einritzen. Erfindungswesentlich ist dabei, daß das System eine Vorrichtung zur aktiven Freigabe des Wirkstoffs in Form einer integrierten Pumpe mit elektronischer Ansteuerung enthält.

Ein wesentlicher Vorteil des erfindungsgemäßen Systems ist es, daß die Hauptbarriere für transdermal applizierte Arzneistoffe - das Stratum corneum - bei dem erfindungsgemäßen System umgangen wird. Gerade die individuell unterschiedlichen Eigenschaften der obersten Hornschicht bei Patienten sind Ursache dafür, daß es bei einer transdermalen Applikation von Wirkstoffen zu Problemen wie mangelnder Bioverfügbarkeit und Allergien kommt. Ein besonderer Vorteil der trancornealen Applikation ist es, daß diese Applikationsart nicht nur auf solche Wirkstoffe beschränkt ist, die durch die Haut penetrieren, wie es beispielsweise bei der transdermaien Applikation der Fall ist. Geeignete Wirkstoffe sind beispielsweise Schmerzmittel - wie z. B. Morphine, Naltrexon, Fentanyl, Oxymorphon; anti-Parkinsonmittel- wie beispielsweise L-Dopa, Pramipexol; Herz-Kreislaufmittel, Nitroglycerin, Mittel gegen Bluthochdruck und vasodilatorische Erkrankungen wie beispielsweise Clonidin, Nifidepine, Verapamil, Diltiazam; Antikoagulantien wie beispielsweise Heparin, Hirudin; Mittel zur Langzeittherapie bei Krebserkrankungen und Immunerkrankungen; Mittel zur Langzeitbehandlung bei der Suchttherapie; Peptide; ACE-Hemmer; Neurokininantagonisten; Hormone - wie beispielsweise Oestradiot.

Üblicherweise liegt der Wirkstoff in Form einer Lösung vor, um einen einwandfreien Transport durch die kapillaren Öffnungen der Mikrostacheln des transcornealen Systems zu gewährleisten. Prinzipiell können alle physiologisch verträglichen Lösungsmittel oder Lösungsmittelgemische, in denen sich der Wirkstoff in ausreichender Menge löst, verwendet werden. Unter einer ausreichenden Menge werden solche Konzentrationen an Wirkstoff im Lösungsmittel verstanden, die es ermöglicht, eine therapeutisch wirksame Menge Wirkstoff applizieren zu können.

Bevorzugte Lösungsmittel sind Wasser und Ethanol. Sollte es notwendig sein, können zur Erhöhung der Löslichkeit des Wirkstoffes im Lösungsmittel Lösungsvermittler und Komplexbildner verwendet werden. Empfindliche Wirkstoffe können zur Erhöhung der Lagerstabilität mit Zusätzen versehen werden.

Das erfindungsgemäße System enthält ein Reservoir zur Speicherung der Wirkstofflösung, wobei eine flüssigkeitsleitende Verbindung zwischen dem Reservoir und den Mikrostacheln es ermöglicht, daß das Arzneimittel aus dem Reservoir durch die kapillaren Öffnungen der Mikrostacheln bis unter das "Stratum comeum" gefördert wird, so daß das Arzneimittel unter Umgehung der äußeren Hautschichten direkt in den Blutkreislauf eingebracht werden kann.

Der Transport des Medikaments - beispielsweise in Form einer wässerigen Lösung-erfolgt erfindungsgemäß durch eine im System integrierte Pumpe mit elektronischer Ansteuenung. Diese Pumpe kann eine piezoelelektrische Membran sein. Der Volumenstrom (ml/Zeit) des Medikaments kann durch ein oder mehrere zusätzliche(s) Ventil(e) oder eine Drosselstrecke zwischen Reservoir und den Mikrostacheln eingestellt, bzw. kontrolliert werden.

In Abhängigkeit der Größe des Reservoirs, der Wirkstoffkonzentration sowie der erforderlichen therapeutischen Dosierung ist das erfindungsgemäße transcorneale System für eine Applikationsdauer von einem über mehreren Tagen bis zu 4 Wochen oder länger, bevorzugt von 7 - 14 Tagen, geeignet.

In einer Ausführungsform ist das System bezüglich seiner Abmessungen und seinem Gewicht so weit miniaturisiert, daß es ohne weiteres über einen längeren Zeitraum auf der Haut bzw. in der Haut fixiert - ähnlich einem Pflaster oder einer Armbanduhr - getragen werden kann. Die Befestigung des transcornealen Systems kann mittels eines Armbandes, einem hautverträglichen Kleber oder auch durch die Mikrostacheln selbst erfolgen.

Die Herstellung des erfindungsgemäßen Systems sowie die Befüllung des Reservoirs erfolgen unter kontrollierten Bedingungen - aus Gründen der Arzneimittelsicherheit kann das erfindungsgemäße System bis zum Gebrauch unter sterilen Bedingungen luftdicht verpackt, bzw. versiegelt sein.

Üblicherweise bilden Reservoir und Mikrostacheln des erfindungsgemäßen Systems eine einteilige oder mehrteilige konstruktive Einheit in einem Gehäuse. Es sind aber Ausführungsformen denkbar, in denen Reservoir und Mikrostachel konstruktiv voneinander getrennt sind und durch einen dünnen Schlauch oder eine Kapillare miteinander verbunden sind. Dies ist besonders dann vorteilhaft, wenn größere Arzneimittelmengen über einen längeren Zeitraum appliziert werden sollen.

Von entscheidender Bedeutung für die Funktion des erfindungsgemäßen transcornealen Systems ist die technische und konstruktive Ausführung der Mikrostacheln, sowie der kapillaren Öffnungen, die zur Zuführung der Wirkstofflösung dienen.

Zur Durchdringung des Stratum comeum ist es erforderlich, daß die Mikrostacheln eine Länge von mindestens 10 µm, bevorzugt 50 - 100 µm, besonders bevorzugt bis zu 1mm aufweisen. Die erfindungsgemäßen Mikrostacheln verlaufen konisch bzw. zylindrisch, wobei die Verrundungsradien der Stachelspitzen typischerweise im µm-Bereich, bevorzugt kleiner als 10 µm sind. Hierdurch wird die Verletzung der Haut und die Schmerzempfindlichkeit bei der Applikation möglichst gering gehalten. Um eine ausreichende Zuführung an Wirkstofflösung in den kapillaren Blutkreislauf des Patienten sicherzustellen, weisen die erfindungsgemäßen Mikrostacheln kapillare Öffnungen, beispielsweise in Form von Bohrungen oder Schlitzen oder einer Kombination von beidem auf. Mikrostacheln aus einem Material definierter Poriosität - ermöglichen ebenfalls eine Zuführung der Wirkstofflösung.

Besondere Ausführungsformen der erfindungsgemäßen Mikrostacheln können beispielsweise kapillare Öffnungen in Form einer Kombination aus einer zentralen Bohrung mit Schlitzen nach außen aufweisen.

Der Transport der Wirkstofflösung kann in Abhängigkeit der Viskosität der Lösung durch mechanische, elektrische, chemische und/oder oberflächenaktive Kräfte unterstützt, bzw. reguliert werden. Aus Gründen der Redundanz - aber auch zur Einstellung des Volumenstroms und des Leitungswiderstandes - kommen pro transcornealem System, bevorzugt eine Vielzahl von Mikrostacheln zum Einsatz. Üblicherweise sind die Mikrostacheln auf einer Fläche angeordnet, die die der Haut zugewandte Seite des transcomealen Systems bildet. Diese Fläche kann zwischen wenigen Quadratmillimetem und einigen Quadratzentimetem betragen. Eine typische Anzahl von Mikrostacheln liegt zwischen 10 und 100, wobei diese Angabe in keiner Weise den Erfindungsgedanken limitieren soll.

Der Wirkstoff, aus dem die Mikrostacheln gefertigt sind, muß hautverträglich und biokompatibel sein. Im Sinne einer kostengünstigen Massenfertigung sind neben keramischen Materialien, Gläser und Metalle - beispielsweise Titan - geeignet. Leicht zu verarbeitende Kunststoffe sind zu bevorzugen. Biologisch abbaubare Polymere - wie z.B. Polylactide und andere - haben den Vorteil, daß eventuell in der Haut zurückbleibende Materialpartikel der Stacheln abgebaut werden können. Bioabbaubare Polymere sind seit längerem aus dem Stand der Technik bekannt und haben sich beispielsweise als Nahtmaterial und Knochenschienen bewährt.

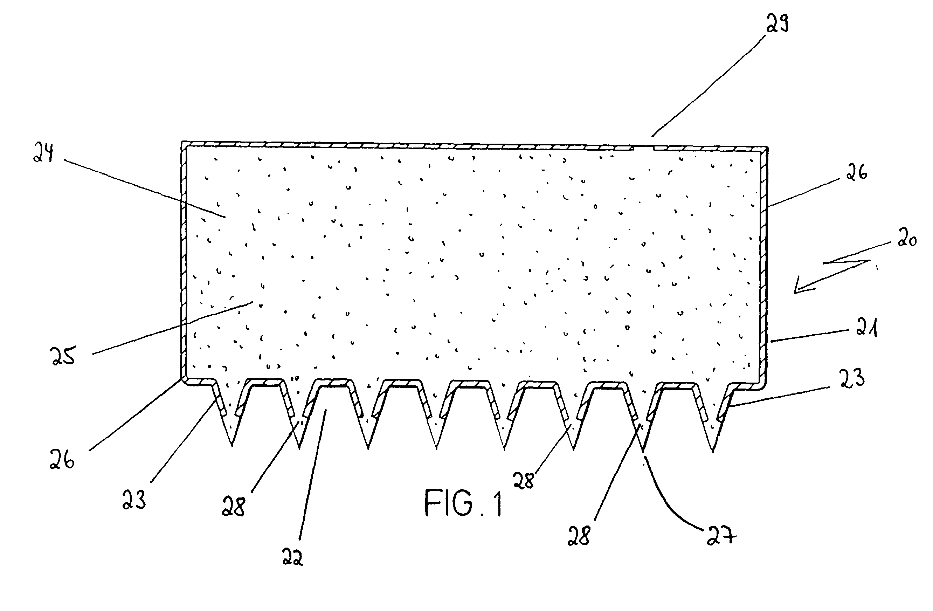

Figur 1 zeigt als Beispiel eine besonders einfache Ausgestaltung eines transcornealen Systems (20) in einem axialen Schnitt. Das System besteht aus einem Behälter (21) mit am Boden (22) angeformten Mikrostacheln (23). Der Innenraum des Behälters dient als Reservoir (24) zur Aufnahme der Wirkstofflösung (25). In Abhängigkeit der Viskosität liegt die Wirkstofflösung als solche direkt im Reservoir vor, oder ist einer Matrix - z.B. aus einem saugfähigen Material oder einem Polymer - gespeichert.

Behälter und Mikrostacheln besitzen eine flüssigkeitsdichte Außenwand (26), die mechanisch so stabil ist, daß das System zur Aktivierung der Arzneimittelfreigabe auf die Haut aufgesetzt und die Mikrostacheln mit leichtem Druck in die Haut gedrückt werden können. Da die Außenwand (26) im Bereich der Spitzen (27) der Mikrostacheln durchbrochen ist und eine Austrittsöffnung (28) bildet, kann die Wirkstofflösung infolge der Kapillarkraft unter Umgehung der transcomealen Hautschicht in das Kapillarkreislaufsystem gelangen und von dort seine systemische Wirkung entfalten. Im Bereich des Reservoirs ist gegebenenfalls eine Einrichtung vorgesehen um einen Druckausgleich - Belüftung (29) - zu schaffen. Üblicherweise ist die Belüftung mit einem Filter versehen, damit Verunreinigungen nicht in das System gelangen können. Erfindungsgemäß ist zur aktiven Freigabe des Wirkstoffs eine integrierte Pumpe mit elektronischer Ansteuerung vorgesehen. Die Befüllung des Systems erfolgt beispielsweise durch Injektion der Wirkstofflösung in das Reservoir, durch Eintauchen des Systems in eine Wirkstofflösung oder durch Einsetzen einer wirkstoffgetränkten Matrix in das System. Es ist selbstverständlich, daß im letztgenannten Fall das transcomeale System zweiteilig aufgebaut ist, beispielsweise aus einem unteren Teil, der die Mikrostacheln bildet und einem oberen Teil, mit dem das System nach Einsetzen der Wirkstoffmatrix verschlossen wird. Je nach Art des Wirkstoffs kann dieser in einem wässerigen oder organischen physiologisch verträglichem Lösungsmittel oder Lösungsmittelgemisch in gelöster Form vorliegen. Geeignete Lösungsmittel sind beispielsweise Wasser, Ethanol, Propanol und deren Gemische. Die Wirkstoffe können jedoch auch in einer Matrix aus einem Gel - beispielsweise aus einem polymeren Material - gelöst sein.

Als Material für die Herstellung des Behälters und der Mikrostacheln kommen in erster Linie Thermoplaste in Frage, die ausgehend von einem feinkörnigen Granulat in einer Form gesintert werden können. Durch geeignete Wahl der Parameter Druck, Temperatur (typischerweise und unterhalb der Schmelztemperatur des Materials) und Zeit wird eine reproduzierbare Porösität (typisch 50 %) eingestellt. Durch anschließendes gezieltes Aufschmelzen der Bauteiloberfläche wird diese verschlossen, so daß ein poröses Behältnis mit dichter Außenwandung entsteht. Wandungsbereiche, die durchlässig gehalten werden sollen, wie z.B. die Entlüftungen, die Stachelspitzen, werden durch Kühlung unterhalb der Schmelztemperatur gehalten. Zum Abdichten der porösen Wandung kommen des weiteren auch Beschichtungen und Versiegelungen in Frage, die aber fertigungstechnisch einen höheren Aufwand bedingen. Grad der Porösität und

Austrittsquerschnitte an den Stachelspitzen sind in gewissen Grenzen variierbar und somit Parameter für die Einstellung der Dosierrate. Andere geeignete Materialien sind beispielsweise Polyethylene, Polypropylene oder Polysulfone.

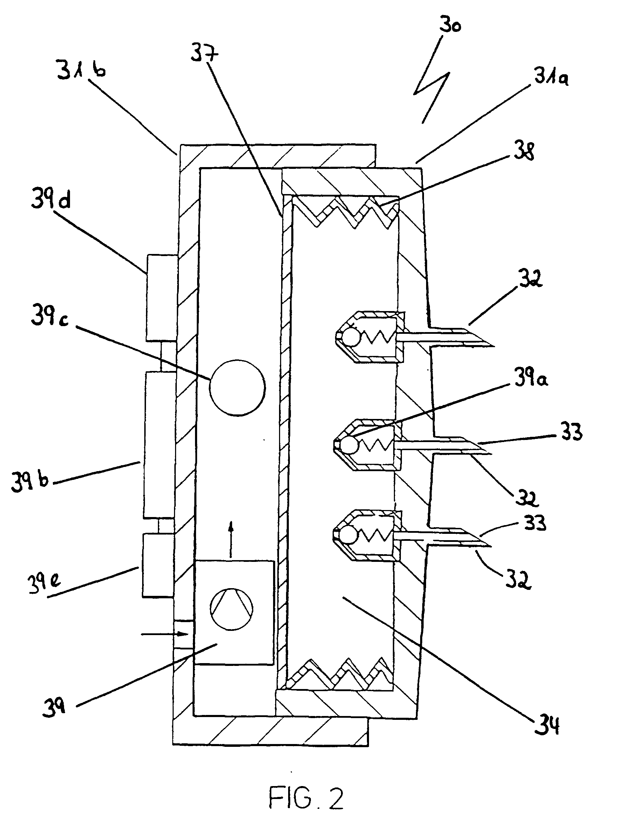

Ein weiterentwickeltes System ist in Figur 2 dargestellt. Das transcomeale System (30) besteht aus einem Gehäuseunterteil (31a) und einem Gehäuseoberteil (31b). Das Gehäuseunterteil (31a) enthält auf der Hautoberfläche zugewandten Seite Mikrostacheln (32) mit den kapillaren Öffnungen (33), von denen in der Zeichnung zur besseren Darstellung nur drei - aber vergrößert - dargestellt sind. Das Reservoir (34) für die Wirkstofflösung wird durch einen beweglichen Kolben (37) und an den Seiten zu dem Gehäuseunterteil durch eine Faltenbalgdichtung (38) gebildet. Die Faltenbalgdichtung kann selbstverständlich durch andere Dichtungsmaßnahmen, beispielsweise eine paßgenaue Führung des Kolbens in dem Gehäuseunterteil, ersetzt werden. Das Gehäuseoberteil enthält die Mikropumpe (39), die einen definierten Druck auf den Kolben ausübt und damit den Wirkstoff durch die Mikrostacheln in das Kapillarkreislaufsystem appliziert. Auf der Innenseite des Gehäuseunterteils können vor den kapillaren Öffnungen Mikroventile (39a) angeordnet sein, um eine vorzeitige Freigabe des Arzneimittels zu verhindern. Der Druck auf den Kolben kann durch die Pumpe pneumatisch erfolgen, kann aber auch in einer anderen Ausführungsform über einen miniaturisierten Elektromotor und ein daran angeschlossenes Getriebe auf rein mechanischem Weg erfolgen. In jedem Fall wird die integrierte Pumpe elektronisch angesteuert.

Zur Verbesserung der Steuer- und Regelbarkeit der Wirkstoffdosierung kann das System um Mikrosensoren (39c), Mikroaktuatoren (39e), z. B. zur aktiven Ansteuerung der Mikroventile (nicht dargestellt), einen elektronischen Schaltkreis (39b) mit Ein-Ausgabe-Möglichkeit (39d) und eine Stromversorgung erweitert werden. Die Sensoren dienen in erster Linie der Erfassung und Überwachung von Regel- und Störgrößen, wie z.B. der Wirkstoffkonzentration im Blut, der Temperatur oder Aktivität des Patienten, und der Erfassung und Überwachung von Systemgrößen, wie z.B. Zeit, Durchfluß, Druck, Temperatur. Der Speicherbereich des elektronischen Schaltkreises ist mit Solldaten und Parametern vom Hersteller oder vom Arzt bzw. Patienten über eine geeignete Schnittstelle programmierbar. Die Meßwerte der Sensoren werden von der Elektronik erfaßt und weiterverarbeitet. Gemäß der vorgegebenen Steuer- und Regelfunktion werden hieraus die Stellsignale für die Mikroaktuatoren abgeleitet.

Ein wesentlicher Bestandteil bei transcomealen Systemen ist die Ausgestaltung der Mikrostacheln. Die Erfindung Liegt hier jedoch im Vorhandensein einer integrierten Pumpe mit elektronischer Ansteuerung zur aktiven Freigabe des Wirkstoffs.

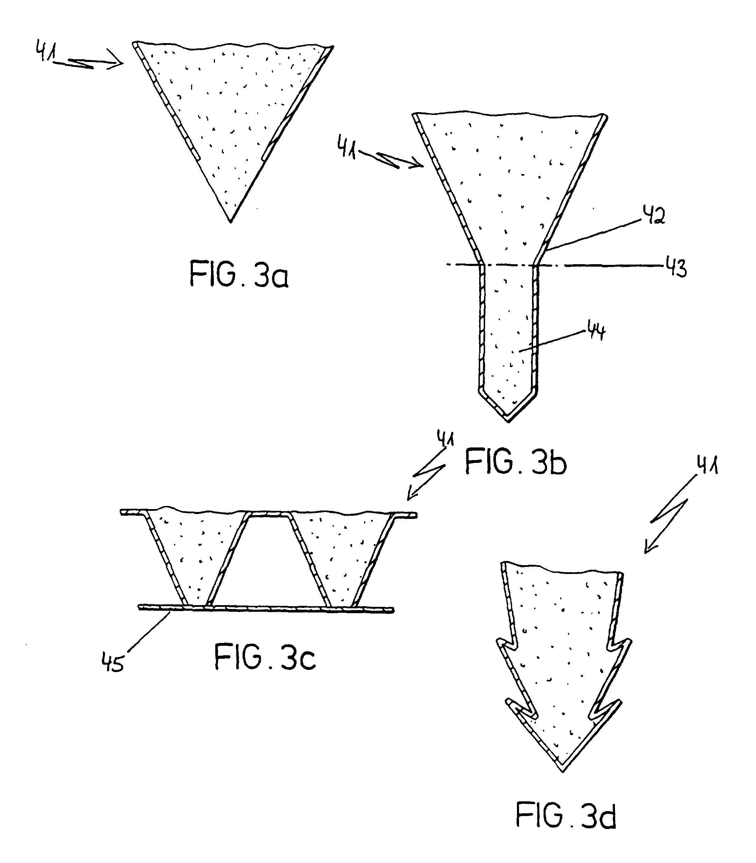

Ausführungsformen von Stacheln (41) sind in Figur 3 dargestellt. Figur 3a zeigt einen an der Spitze porös und damit für die Wirkstofflösung durchgängig gehaltenen Stachel (41). Figur 3b ist ein Stachel (42) mit einer vollständig dichten Außenwandung dargestellt. Die Spitze besitzt einen Fortsatz (44), der beim Einstechen an seiner Wurzel, der konstruierten Sollbruchstelle (43), abbricht und damit an der Bruchstelle die zuvor dichte Stachelspitze öffnet. Eine andere Möglichkeit der Öffnung der Stachelspitzen besteht darin, die durch eine Siegelfolie (45) zunächst verschlossenen Domspitzen bei Anbruch abzuziehen und damit die Stachelspitzen "aufzureißen" (Fig. 3c). Zur Verankerung des transcornealen Systems können Widerhaken an den Stacheln angeformt werden, s. Fig. 3d. Die Stacheln sind grundsätzlich aus einem biologisch verträglichem Material gefertigt, wie beispielsweise Metalle, Keramiken und Polymere, wie beispielsweise bioabbaubare Polymere auf der Basis von Glykolid und/oder Laktid, möglicherweise als Copolymere mit anderen biologisch abbaubaren Polymeren. Die Stacheln können auch aus einem porösen, für den Wirkstoff durchlässigem Material gefertigt sein, beispielsweise thermoplastische Kunststoffe, so daß der Wirkstoff über die gesamte Fläche der Stacheln abgegeben wird.

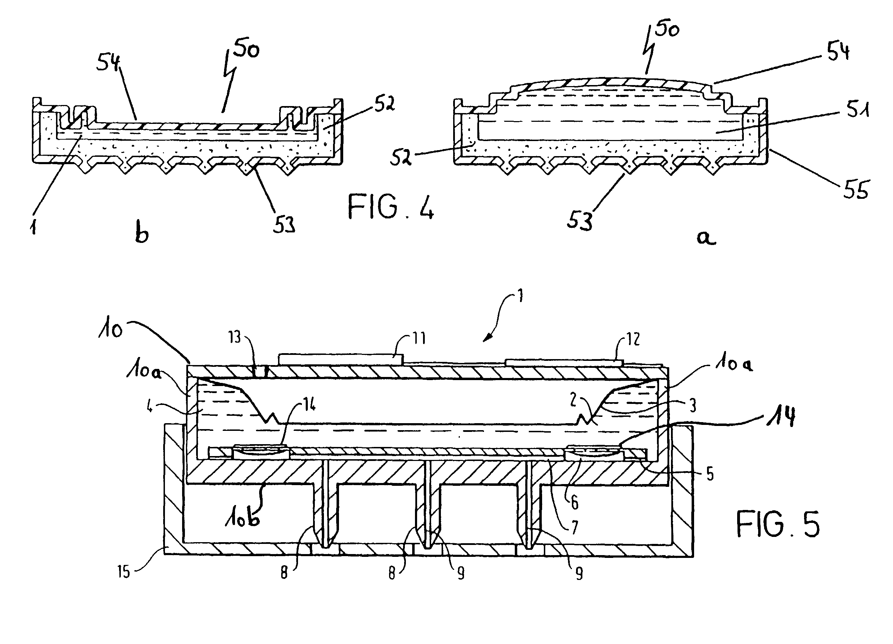

Figur 4 zeigt ein wannenartig geformtes Reservoir (50), bei dem die Wirkstofflösung (51) mit einer elastischen Membran (54) nach außen abgeschlossen ist. Je nach Ausführungsform des transcornealen Systems bilden das Reservoir und die in die Haut eindringenden Mikrostacheln (53) eine konstruktive Einheit. Die Reservoirwandung (55) und die Stacheln (53) sind wie oben beschrieben aus einem porösen Material, deren außenliegende Oberfläche abgedichtet ist. Die Wirkstofflösung wird unter leichtem Überdruck in Wirkstoffmatrix (52) injiziert. Der Überdruck wird von der elastischen Membran (54) gehalten und steht somit zur Konstanthaltung der Durchflußrate zur Verfügung. Der Durchfluß kann von außen (Patient) durch Drücken der Membran auch kurzzeitig erhöht werden, um eine zusätzliche Dosis zu ermöglichen. Figur 4a zeigt das erfindungsgemäße System im Anfangszustand, die nach außen gewölbte Membran (54) sorgt dafür, daß die Wirkstofflösung unter Druck steht und in das Wirkstoffreservoir (52) gedrückt wird. Der Wirkstoff gelangt über die Mikrostacheln (53) durch die transcomeale Hautschicht zur Erzielung einer Systemischen Wirkung.

Figur 4b zeigt die Membran (54) nachdem ein Großteil der Wirkstofflösung aufgebraucht ist.

Figur 5 zeigt einen Schnitt durch ein transcorneales System (1). Das Gehäuse (10) enthält ein Wirkstoffreservoir (2), das an seiner Oberseite durch einen Faltenbalg (3) abgeschlossen ist. In dem Wirkstoffreservoir befindet sich die Wirkstofflösung (4), die an der unteren Seite des Wirkstoffreservoirs über einen Einlaßkanal (5) in eine Pumpkammer (6) gelangt. Über einen Auslaßkanal (7) gelangt die Wirkstofflösung zu den an der Unterseite des Gehäuses angeordnete Mikrostacheln (8) und von dort durch die kapillaren Öffnungen (9) der Mikrostacheln nach außen. Die Gehäuseseitenteile (10a) und die Gehäuseunterseite (10b) bilden zusammen mit den Mikrostacheln eine konstruktive Einheit, bevorzugt aus einen thermoplastischen Kunststoff. Der Deckel des Gehäuses enthält die Energieversorgung in Form einer Batterie (11) sowie eine elektronische Steuerung (12), eine Belüftung (13) ermöglicht, daß der Faltenbalg bei der Abgabe von Wirkstofflösung durch die Mikrostacheln sich diesem verringerten Volumen anpassen kann. Die Förderung der Wirkstofflösung erfolgt durch eine piezoelektrische Membran (14), die eine elektrisch angesteuerte Pumpbewegung ausführt. Der Einlaßkanal (5) ist derart gestaltet, daß die Wirkstofflösung durch die piezoelektrische Membran (14) zu den Auslässen der Mikrostacheln gepumpt wird. Dies erfolgt entweder durch ein Ventil oder dadurch daß der Querschnitt des Einlaßkanals geringer als der des Auslaßkanals (7) ist. Vor dem Gebrauch des transcornealen Systems sind die Mikrostacheln durch einen Stachelschutz (15), beispielsweise in Form einer Kappe, geschützt.

Figur 6 zeigt einige Ausführungsformen von im erfindungsgemäßen System eingesetzten Mikrostecheln in einem Schnitt und einer Draufsicht.

Figur 6a zeigt einen Mikrostachel mit einer zentralen Öffnung (9) und zylindrischer Außenform (8) und einem konischen Spitze (10).

Figur 6b zeigt einen Mikrostachel mit einer Öffnung in Form einers Schlitzes (9) und zylindrischer Außenform (8).

Figur 6c zeigt einen Mikrostachel mit abgeflachten Außenseiten (8), wobei die Öffnung in Form eines Schlitzes angeordnet ist.

Figur 6d zeigt einen Mikrostachel mit zylindrischer Außenform und schräger Spitze (10).

Figur 6e zeigt eine Ausführungsform von Mikroschneiden, die anstelle der Mikrostacheln verwendet werden können in einem Schnitt und einer Draufsicht.

Die Öffnungen (9) für die Wirkstofflösung befindet sich überlicherweise dicht neben der Schneide (8a) an der Unterseite (10b) des Reservoirs (siehe Figur 5), so daß die Wirkstofflösung von dort durch die geritzte Hautoberfläche gelangt und ihre systemische Wirkung entfalten kann.

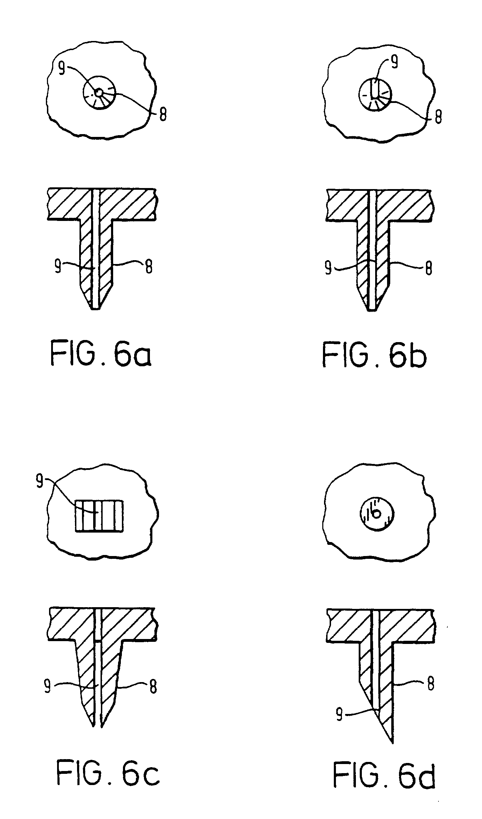

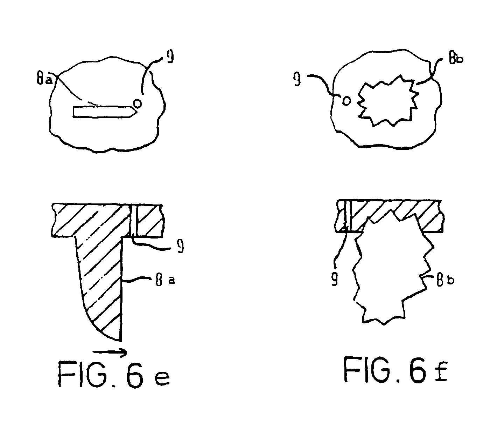

Figur 6f zeigt eine Ausführungsform einer Mikroschneide in Form eines Kornes mit spitzen - die Haut ritzenden - Kanten (8b). Die Öffnung(en) (9) befinden sich im näheren Bereich des Korns.

Die Abmessungen der Mikroschneiden haben in etwa dieselbe Größenordnung wie die bereits beschriebenen Mikrostacheln.

Die einzelnen Mikrostacheln bzw. Mikroschneiden sind typischerweise auf der Unterseite des transcomealen Systems angeordnet und bilden eine konstruktive Einheit; ihre Anzahl beträgt beispielsweise zwischen 10 und 100.

Die Dosierung des Arzneimittels kann über die Volumenströme gesteuert werden, die wiederum von der Summe der Querschnitte der Öffnungen der Mikrostacheln abhängig ist.

## Patentansprüche

1. Transcorneales System (1) zur kontrollierten Freigabe von Arzneimitteln enthaltend
- ein Wirkstoffreservoir (2),
- eine Vorrichtung mit Mikrostacheln (8) mit kapillaren Öffnungen (9) oder Mikroschneiden (8a) die eine Länge von mindestens 10 µm aufweisen und die über eine Flüssigkeit-leitende Verbindung so mit dem Wirkstoffreservoir verbunden sind, daß ein aktiver Transport des Wirkstoffs aus dem Reservoir durch die Mikrostacheln oder entlang den Mikroschneiden möglich ist,
- eine Vorrichtung zur aktiven Freigabe des Wirkstoffs aus dem Reservoir durch die Mikrostacheln oder entlang den Mikroschneiden,
dadurch charakterisiert, daß
- die Vorrichtung zur aktiven Freigabe des Wirkstoffs eine integrierte Pumpe (14,39) mit elektronischer Ansteuerung (12) ist.

2. Transcorneales System nach Anspruch 1, dadurch charakterisiert, daß
- Mikroventile (39a) zwischen dem Reservoir und den kapillaren Öffnungen der Mikrostacheln angeordnet sind .

3. Transcorneales System nach einem der Ansprüche 1 oder 2, dadurch charakterisiert, daß
- jeder Mikrostachel eine kapillare Öffnung (9) in Form einer zentralen Bohrung oder eines Schlitzes aufweist, die an ihrer Spitze in einer Austrittsöffnung mündet und diese mit dem Wirsktoffreservoir verbindet.

4. Transcorneales System nach einem der Ansprüche 2 oder 3, dadurch charakterisiert, daß
- die Mikrostacheln aus einem porösen, flüssigkeitsdurchlässigen Material zum Einstellen der Dosierrate bestehen .

5. Transcorneales System nach einem der Ansprüche 1 bis 4, dadurch charakterisiert, daß
- die integrierte Pumpe eine elektrisch angesteuerte piezoelektrische Membran ist.

6. Transcorneales System nach einem der Ansprüche 1 bis 5, dadurch charakterisiert, daß
- die integrierte Pumpe eine Mikropumpe (39) ist.

7. Transcorneales System nach einem der Ansprüche 1 bis 6, dadurch charakterisiert, daß
- wenigstens eine Begrenzungsfläche des Reservoirs beweglich gestaltet ist .

8. Transcorneales System nach Anspruch 7, dadurch charakterisiert, daß
- die bewegliche Begrenzungsfläche ein Kolben (37) ist.

9. Transcorneales System nach Anspruch 7, dadurch charakterisiert, daß
- die bewegliche Begrenzungsfläche eine Membran (54) ist.

10. Transcorneales System nach Anspruch 7, dadurch charakterisiert, daß
- die bewegliche Begrenzungsfläche eine Faltenbalgdichtung (3) ist.

11. Transcorneales System nach einem der Ansprüche 1 bis 10, dadurch charakterisiert, daß
- eine Einrichtung zum Druckausgleich ausgebildet ist .

12. Transcorneales System nach einem der Ansprüche 1 bis 11, dadurch charakterisiert, daß
- die Mikrostacheln integrale Bestandteile des Reservoirs sind .

13. Transcorneales System nach einem der Ansprüche 1 bis 12, dadurch charakterisiert, daß
- die Mikrostacheln oder Mikroschneiden aus einem thermoplastischen Kunststoff bestehen .

14. Transcorneales System nach einem der Ansprüche 1 bis 13, dadurch charakterisiert, daß
- die Mikrostacheln oder Mikroschneiden eine Spitze mit einem Krümmungsradius von weniger als 10 µm aufweisen .

15. Transcorneales System nach einem der Ansprüche 1 bis 14, dadurch charakterisiert, daß
- der Wirkstoff ein systemisch wirkendes Arzneimittel ist .

16. Transcorneales System nach einem der Ansprüche 1 bis 15, dadurch charakterisiert, daß
- der Wirkstoff in Form einer Lösung in dem Reservoir vorliegt .

## Claims

1. Transcorneal system (1) for the controlled release of drugs, comprising
- an active substance reservoir (2),
- a device with micro-pins (8) having capillary openings (9) or micro-blades (8a) which are at least 10 *µ* m long and are connected to the reservoir of active substance via a liquid-conveying connection in such a way that the active substance can be actively transported from the reservoir through the micro-pins or along the micro-blades,
- a device for actively releasing the active substance from the reservoir through the micro-pins or along the micro-blades,
**characterised in that**
- the device for actively releasing the active substance is an integrated pump (14, 39) with electronic actuating means (12).

2. Transcorneal system according to claim 1,
**characterised in that**
- microvalves (39a) are provided between the reservoir and the capillary openings of the micro-pins.

3. Transcorneal system according to one of claims 1 and 2, **characterised in that**
- each micro-pin has a capillary opening (9) in the form of a central bore or a slot which terminates at its tip in an outlet opening and connects the latter to the active substance reservoir.

4. Transcorneal system according to one of claims 2 and 3, **characterised in that**
- the micro-pins consist of a porous, fluid-pervious material for adjusting the dosage rate.

5. Transcorneal system according to one of claims 1 to 4, **characterised in that**
- the integrated pump is an electrically actuated piezoelectric membrane.

6. Transcorneal system according to one of claims 1 to 5, **characterised in that**
- the integrated pump is a micropump (39).

7. Transcorneal system according to one of claims 1 to 6, **characterised in that**
- at least one boundary surface of the reservoir is of movable construction.

8. Transcorneal system according to claim 7,
**characterised in that**
- the movable boundary surface is a plunger (37).

9. Transcorneal system according to claim 7,
**characterised in that**
- the movable boundary surface is a membrane (54).

10. Transcorneal system according to claim 7,
**characterised in that**
- the movable boundary surface is a concertina seal (3).

11. Transcorneal system according to one of claims 1 to 10, **characterised in that**
- means for pressure equalisation are provided.

12. Transcorneal system according to one of claims 1 to 11, **characterised in that**
- the micro-pins are integral components of the reservoir.

13. Transcorneal system according to one of claims 1 to 12, **characterised in that**
- the micro-pins or micro-blades consist of a thermoplastic material.

14. Transcorneal system according to one of claims 1 to 13, **characterised in that**
- the micro-pins or micro-blades have a tip with a radius of curvature of less than 10 *µ*m.

15. Transcorneal system according to one of claims 1 to 14, **characterised in that**
- the active substance is a systemically acting medicament.

16. Transcorneal system according to one of claims 1 to 15, **characterised in that**
- the active substance is present in the reservoir in the form of a solution.

## Revendications

1. Système transcornéen (1) pour la libération contrôlée de médicaments contenant
- un réservoir de principe actif (2),
- un dispositif avec des micropointes (8) à ouvertures capillaires (9) ou des microlames (8a) qui présentent une longueur d'au moins 10 *µ*m et qui sont reliées au réservoir de principe actif par une liaison conduisant les liquides de telle manière qu'un transport actif du principe actif depuis le réservoir par les micropointes ou le long des microlames est possible,
- un dispositif pour la libération active du principe actif depuis le réservoir par les micropointes ou le long des microlames,
**caractérisé en ce que**
- le dispositif pour la libération active du principe actif est une pompe intégrée (14, 39) à commande électronique (12).

2. Système transcornéen selon la revendication 1 **caractérisé en ce que**
- des microvannes (39a) sont disposées entre le réservoir et les ouvertures capillaires des micropointes.

3. Système transcornéen selon l'une des revendications 1 et 2 **caractérisé en ce que**
- chaque micropointe présente une ouverture capillaire (9) sous forme d'un trou central ou d'une fente qui débouche à son extrémité dans une ouverture de sortie et qui relie celle-ci au réservoir de principe actif.

4. Système transcornéen selon l'une des revendications 2 et 3 **caractérisé en ce que**
- les micropointes consistent en un matériau poreux perméable aux liquides pour ajuster le dosage.

5. Système transcornéen selon l'une des revendications 1 à 4 **caractérisé en ce que**
- la pompe intégrée est une membrane piézo-électrique commandée électriquement.

6. Système transcornéen selon l'une des revendications 1 à 5 **caractérisé en ce que**
- la pompe intégrée est une micropompe (39).

7. Système transcornéen selon l'une des revendications 1 à 6 **caractérisé en ce que**
- au moins une surface de limitation du réservoir est mobile.

8. Système transcornéen selon la revendication 7 **caractérisé en ce que**
- la surface de limitation mobile est un piston (37).

9. Système transcornéen selon la revendication 7 **caractérisé en ce que**
- la surface de limitation mobile est une membrane (54).

10. Système transcornéen selon la revendication 7 **caractérisé en ce que**
- la surface de limitation mobile est un élément d'étanchéité à soufflet (3).

11. Système transcornéen selon l'une des revendications 1 à 10 **caractérisé en ce que**
- un dispositif est prévu pour l'égalisation de pression.

12. Système transcornéen selon l'une des revendications 1 à 11 **caractérisé en ce que**
- les micropointes font partie intégrante du réservoir.

13. Système transcornéen selon l'une des revendications 1 à 12 **caractérisé en ce que**
- les micropointes ou les microlames consistent en une matière synthétique thermoplastique.

14. Système transcornéen selon l'une des revendications 1 à 13 **caractérisé en ce que**
- les micropointes ou les microlames présentent une extrémité ayant un rayon de courbure inférieur à 10 µm.

15. Système transcornéen selon l'une des revendications 1 à 14 **caractérisé en ce que**
- le principe actif est un médicament à action systémique.

16. Système transcornéen selon l'une des revendications 1 à 15 **caractérisé en ce que**
- le principe actif est sous forme d'une solution dans le réservoir.
